# EUROPEAN PATENT APPLICATION

(11) **EP 4 156 197 A1**
(43) Date of publication of application: **29.03.2023**
(21) Application number: 21199029.6
(22) Date of filing: 27.09.2021
(51) Int. Cl.: G16H 20/40, G16H 50/20, G16H 50/70, G16H 40/63, G16H 40/67

(54) **SYSTEM AND METHOD FOR PROGRAMMING AN ACTIVE MEDICAL IMPLANT BY MEANS OF AN EXTERNAL PROGRAMMING DEVICE**

(71) Applicant: BIOTRONIK SE & Co. KG, 12359 Berlin (DE)
(72) Inventor: MÜLLER, Jens, 14195 Berlin (DE); TIETZ, Marko, 10243 Berlin (DE); DEUTSCHMANN, Hendrik, 12435 Berlin (DE)
(74) Representative: Biotronik Corporate Services SE

(57) **Abstract**

The present disclosure provides A system (1) for programming an active medical implant (2) by means of a programming device (3), comprising: a programming device (3) configured for programming an active medical implant (2); and at least one server (4), wherein the programming device (3) is configured to communicate with the at least one server (4) and to transmit to the server (4) data (D1) requested from the implant (2) and/or information (D3) provided by a user (P) regarding a patient and/or the implant (2), and wherein the at least one server (4) includes a recommendation module (4a) configured to determine a recommended course of action for the user (P) using an artificial intelligence algorithm based on said data (D1) and/or information (D3), wherein the programming device (3) is configured to output the recommended course of action to the user (P).

## Description

Embodiments of the present disclosure relate to a system for programming an active medical implant by means of an external programming device, a method for programming an active medical implant by means of an external programming device, and a machine-readable medium for executing the method. Embodiments of the present disclosure relate more particularly to a remote programming of active medical implants using artificial intelligence.

Medical implants are widely used to replace, support and/or enhance biological structures of patients. Examples of medical implants include, but are not limited to, cardiovascular medical devices such as artificial hearts, artificial heart valves, implantable cardioverter-defibrillators, cardiac pacemakers, and coronary stents. The medical implants can be programmed by users, such as medical personnel, using dedicated programming devices.

Currently, remote support solutions for implantation and follow-up of medical implants are implemented and introduced. For this remote support there are specific employees, such as remote supporters. The remote supporter is in telephone contact with the user of the programming device and can remotely access (screen-share) and operate (remote control) the user interface of the programming device. The remote supporter's advice essentially refers to the information displayed on the interface of the programming device and the verbal information provided by the remote support requestor.

Such remote support solutions require personnel, are expensive and complicate workflows in a clinic.

In light of the above, a system for programming an active medical implant by means of an external programming device, a method for programming an active medical implant by means of an external programming device, and a machine-readable medium for executing the method that overcome at least some of the problems in the art are beneficial.

It is an object of the present disclosure to provide a system for programming an active medical implant by means of an external programming device, a method for programming an active medical implant by means of an external programming device, and a machine-readable medium for executing the method that can facilitate the programming of an active medical implant for the user, in particular a physician.

The objects are solved by the features of the independent claims. Preferred embodiments are defined in the dependent claims.

According to an independent aspect of the present disclosure, a system for programming an active medical implant by means of a programming device is provided. The system includes a programming device configured for programming an active medical implant and at least one server. The programming device is configured to communicate with the at least one server and to transmit to the server data requested from the implant and/or information provided by a user regarding a patient and/or the implant. The at least one server includes a recommendation module configured to provide a recommended course of action for the user using an artificial intelligence algorithm based on said data and/or information. The programming device is configured to output the recommended course of action to the user.

Preferably, the user belongs to medical staff. In particular, the user may be a physician.

According to some embodiments, which can be combined with other embodiments described herein, the artificial intelligence algorithm is a machine learning algorithm. For example, the recommendation module may implement a neural network to analyze the data / information in order to determine the recommended course of action which is output to the user.

The term "artificial intelligence" as used throughout the present application may be understood in the sense of software components or software instances which are designed to correctly interpret data, to learn from such data, and to use those learnings to determine the recommended course of action through flexible adaptation.

The term "machine learning algorithm" as used throughout the present application refers to a software algorithm that can build a model based on training data, in order to make predictions and/or decisions without being explicitly programmed to do so.

In some embodiments, a deep learning architecture is used to train a neural network. Preferably, the neural network is a feed forward network with multiple hidden layers or a recurrent neural network with multiple hidden layers. If the procedure requires, among other things, the processing of image data, video data, or audio data, it is preferable to use a neural network with more than 2 dimensions (e.g., a convolutional neural network with 3 dimensions).

Preferably, the artificial intelligence algorithm is configured to perform input signal conditioning (e.g., filtering, scaling, normalization, transformation, and the like) and/or result post-processing (e.g., clustering, weighting, filtering, plausibility checking, and the like).

Additionally, or alternatively, a data and/or model control layer can be implemented to make the medical application traceable (verification and monitoring of the model). The type of data used to train the neural network may depend on the desired medical support function. In any case, preprocessed data can be used for training the neural network (e.g., anonymous training).

According to some embodiments, which can be combined with other embodiments described herein, the data requested by the programming device from the active medical implant may include measurement data and/or one or more operational parameters.

Preferably, the measurement data are selected from the group including (or consisting of) impedances, shock impedances, sensitivity, stimulus thresholds, temperature, event-based intracardiac recordings, non-event-based intracardiac recordings, and alarms.

Additionally, or alternatively, the one or more operational parameters are selected from the group including (or consisting of) programmed parameters, implant settings, and technical parameters of the active medical implant.

According to some embodiments, which can be combined with other embodiments described herein, the information regarding the patient and/or the active medical implant may be selected from the group including (or consisting of) data collected by the user (e.g., during the pre-implantation consultation), data provided by the patient, and relevant events concerning the patient.

Preferably, the data collected by the user is selected from the group including (or consisting of) main indication, sub-indications, diagnosis, patient history, medication, laboratory results, and medical assessments.

Preferably, the data provided by the patient is selected from the group including (or consisting of) sensor data (e.g. personal health data, for instance from wearables such as smart watches) and personal preferences of the patient.

Preferably, the relevant events concerning the patient are selected from the group including (or consisting of) complaints, studies, new medical findings, and best practice.

According to some embodiments, which can be combined with other embodiments described herein, the programming device includes a user interface. For example, the user interface may include, or be, a touch user interface and/or a voice user interface.

Preferably, the user interface is configured to output the recommended course of action to the user, e.g., by displaying information regarding the recommended course of action and/or outputting audio information regarding the recommended course of action.

According to some embodiments, which can be combined with other embodiments described herein, the user interface is configured to receive a user input from the user confirming the recommended course of action.

Preferably, the programming device is configured to automatically execute the recommended course of action in response to the confirmation received at the user interface.

According to some embodiments, which can be combined with other embodiments described herein, the recommended course of action includes a recommended programming of the active medical implant. For example, the programming device may be configured to automatically program the active medical implant according to the recommended programming provided by the at least one server in response to the confirmation received at the user interface.

According to some embodiments, which can be combined with other embodiments described herein, the programming device and the at least one server are connected via at least one communications network.

Preferably, the at least one communications network includes a local area network and/or a wide area network. The at least one communication network is preferably connected to/with the internet.

Preferably, the wide area network is configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), Fifth Generation Technology Standard (5G), and/or any other or future cellular/mobile network.

According to some embodiments, which can be combined with other embodiments described herein, the programming device and the at least one server are configured for encrypted communication, in particular by means of at least one of symmetric encryption, asymmetric encryption, hashes, and signatures.

According to some embodiments, which can be combined with other embodiments described herein, the at least one server is configured to receive support data from at least one external data source.

Preferably, the recommendation module is configured to determine the recommended course of action further based on the support data received from the at least one external data source.

Preferably, the support data are selected from the group including (or consisting of) medical data, anonymous patient data (e.g., from other clinics/regions/countries), and third-party information (e.g., weather, lifestyle, etc.). The medical data may be selected from the group including (or consisting of) main indication, sub-indications, diagnosis, patient history, medication, laboratory results, and medical assessments.

Preferably, the at least one external data source includes an EHR (electronic health record) system. The EHR system may provide at least a part of the medical data used to determine the recommended course of action.

According to some embodiments, which can be combined with other embodiments described herein, the system is configured for programming the active medical implant during an implantation of the implant and/or a follow-up session after the implantation.

According to some embodiments, which can be combined with other embodiments described herein, the programming device is a stationary or portable medical support device for medical personnel.

Preferably, the portable medical support device is a mobile terminal. The mobile terminal may be selected from the group including (or consisting of) a personal computer, a tablet, a mobile/smart phone, and a notebook.

According to some embodiments, which can be combined with other embodiments described herein, the active medical implant is configured for cardiac rhythm management or spinal cord stimulation.

According to another independent aspect of the present disclosure, a method for programming an active medical implant by means of a programming device is provided. The method includes receiving, at a programming device configured for programming an active medical implant, data from the implant and/or information provided by a user regarding the implant; receiving, at a server, the data from the implant and/or the information from the programming device; determining, by the server, a recommended course of action for the user using an artificial intelligence algorithm based on said data and/or information; and outputting, by the programming device, the recommended course of action to the user.

Embodiments are also directed at systems/devices for carrying out the disclosed methods and include system/device aspects for performing each described method aspect. These method aspects may be performed by way of hardware components, a computer programmed by appropriate software, by any combination of the two or in any other manner. Furthermore, embodiments according to the invention are also directed at methods for operating the described device/system. It includes method aspects for carrying out every function of the device/system.

According to another independent aspect of the present disclosure, a machine-readable medium is provided. The machine-readable medium includes instructions executable by processors in order to implement the method for programming an active medical implant by means of a programming device of the embodiments of the present disclosure.

The (e.g. non-transitory) machine readable medium may include, for example, optical media such as CD-ROMs and digital video disks (DVDs), and semiconductor memory devices such as Electrically Programmable Read-Only Memory (EPROM), and Electrically Erasable Programmable Read-Only Memory (EEPROM). The machine-readable medium may be used to tangibly retain computer program instructions or code organized into one or more modules and written in any desired computer programming language. When executed by, for example, one or more processors such computer program code may implement one or more of the methods described herein.

So that the manner in which the above recited features of the present disclosure can be understood in detail, a more particular description of the disclosure, briefly summarized above, may be had by reference to embodiments. The accompanying drawings relate to embodiments of the disclosure and are described in the following:

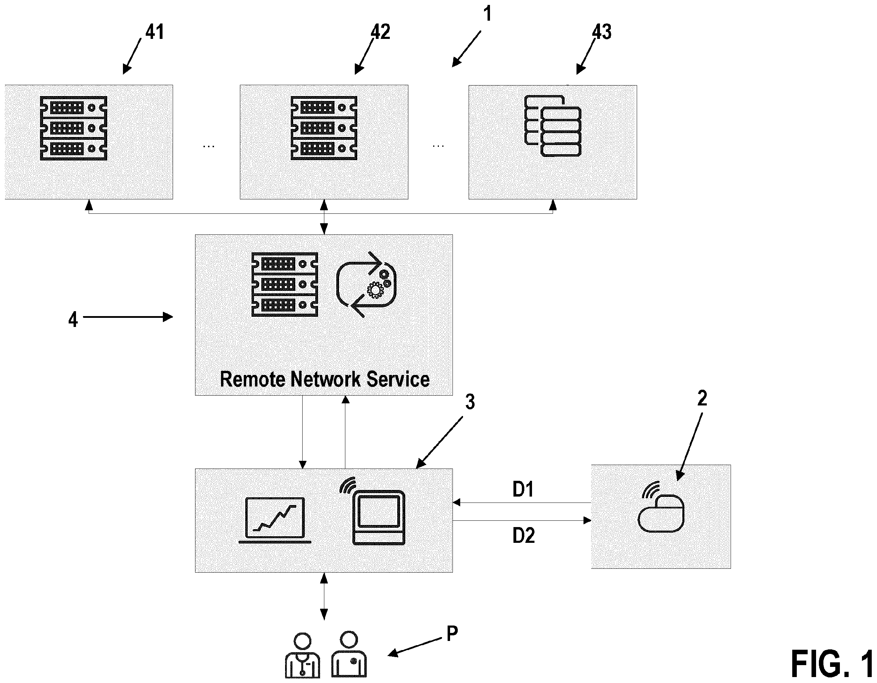

- FIG. 1: shows a schematic view of a system for programming an active medical implant by means of a programming device according to embodiments of the present disclosure.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

Currently, remote support solutions for implantation and follow-up of medical implants are implemented and introduced. For this remote support there are specific employees, such as remote supporters. The remote supporter is in telephone contact with the user of the programming device and can remotely access (screen-share) and operate (remote control) the user interface of the programming device. The remote supporter's advice essentially refers to the information displayed on the interface of the programming device and the verbal information provided by the remote support requestor. Such remote support solutions require personnel, are expensive and complicate workflows e.g. in a clinic.

According to the embodiments of the present disclosure, during the implantation and/or follow-up of active medical implants by means of an external programming device, the user (e.g., medical staff, field rep, etc.) can use an AI-based remote service for assistance. For this purpose, implant data and/or additional information of the user are sent to a server of a remote network service (RNS) via the external programming device. In some embodiments, the RNS may take into account further data sources that may be of interest when assisting the user. The RNS calculates and defines a recommendation for the user by using AI. The recommendation is output by means of the external programming device. The user can then decide whether to follow the recommendation or to continue with the device check and/or manual program creation.

Thereby, a workflow in a clinic can be optimized, less personnel such as remote tech support or field service is required, therapy quality and individual care can be improved, and healthcare costs can be reduced. Furthermore, hardware changes to existing implants are not necessary.

FIG. 1 shows a schematic view of a system 1 for programming an active medical implant 2 by means of a programming device 3 according to embodiments of the present disclosure. The system 1 includes the programming device 3 configured for programming the active medical implant 2 and at least one server 4. The programming device 3 is configured to communicate with the at least one server 4 and to transmit to the server 4 data D1 requested from the implant 2 and/or information D3 provided by a user P regarding a patient and/or the implant 2. The at least one server 4 includes a recommendation module 4a configured to provide a recommended course of action for the user P using an artificial intelligence algorithm based on said data D1 and/or information D3. The programming device 3 is configured to output the recommended course of action to the user P.

In some embodiments, the active medical implant 2 may be configured for cardiac rhythm management or spinal cord stimulation. Examples of medical implants 2 include, but are not limited to, cardiovascular medical devices such as artificial hearts, artificial heart valves, implantable cardioverter-defibrillators, cardiac pacemakers, and coronary stents.

The programming device 3 is configured to program the active medical implant 2. In particular, the programming device 3 may send program instructions and/or program code to the active medical implant 2 which define operational characteristics of the active medical implant 2.

The active medical implant 2 and the programming device 3 may be configured to communicate with each other via a communication connection 10. The communication connection 10 may use any of various wired and/or wireless communication technologies, such as Local Area Networks (LANs), Wireless LAN (WiFi), Bluetooth, and the like.

The communication connection 10 is configured for bidirectional communication. For example, the programming device 3 may request data D1 from the implant and may optionally transmit program instructions D2 and/or program code D2 to the active medical implant 2.

In some embodiments, the data D1 requested from the implant 2 may include measurement data and/or operational parameters. The measurement data may be selected from the group including impedances, shock impedances, sensitivity, stimulus thresholds, temperature, event-based intracardiac recordings, non-event-based intracardiac recordings, and alarms. The operational parameters may be selected from the group including programmed parameters, implant settings, and technical parameters. However, the present disclosure is not limited thereto and the data D1 requested from the implant 2 may include other data suitable to determine the recommended curse of action.

In some embodiments, the programming device 3 is configured to receive information D3 provided from the user P regarding the patient and/or the implant 2. For example, the user P may provide the information D3 using a user interface of the programming device 3, such as a touch user interface and/or a voice user interface. For example, the user interface may include a touch screen configured to receive a touch input from the user P. Additionally, or alternatively, the user interface may be configured to receive a voice input from the user P.

In some embodiments, the information D3 regarding the patient and/or the implant 2 may be selected from the group including data collected by the user P (e.g., during the pre-implantation consultation), data provided by the patient, and relevant events concerning the patient. The data collected by the user P may be selected from the group including main indication, sub-indications, diagnosis, patient history, medication, laboratory results, and medical assessments. The data provided by the patient may be selected from the group including sensor data (e.g. personal health data, for instance from smart watches) and personal preferences of the patient. The relevant events concerning the patient may be selected from the group including complaints, studies, new medical findings, and best practice.

However, the present disclosure is not limited to the above examples and the information D3 regarding the patient and/or the implant 2 may include other information suitable to determine the recommended curse of action, such as personal preferences of the patient's relatives and/or medical staff as well as infrastructure information of the patient's home or residence.

The programming device 3 and the at least one server 4 may be configured to communicate with each other via at least one communications network 11. In particular, the programming device 3 may be configured to transmit to the at least one server 4 the data D1 and/or the information D3 via the at least one communications network 11. Furthermore, the at least one server 4 may be configured to transmit the recommended course of action to the programming device 3 via the at least one communications network 11.

Preferably, the at least one communications network 11 includes a local area network and/or a wide area network. For example, the wide area network may be configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), and Fifth Generation Technology Standard (5G).

In some embodiments, the at least one communications network 11 may be configured for extremely low-latency communication. In addition to the security mechanisms offered by, for example, 5G or broadband network technology, the transmitted information units may be additionally protected against falsification and misuse e.g. at a business layer level. For example, symmetric encryption (AES, RC5, etc.), asymmetric encryption (RSA, etc.), hashes (CMAC, etc.), signatures (DSS, etc.) and/or comparable procedures can be used.

Communication between the programming device 3 and the at least one server 4 can be direct or indirect using, for example, intermediary cloud infrastructures. Due to regional regulatory requirements, the remote network service can be operated in a decentralized manner by means of scalable real-time capable Internet of Things (IoT) infrastructures (cloud services, SaaS, etc.). Therefore, it is possible for external programmers in one country, for example, to communicate with a locally operated remote network services, while external programmers in other countries access remote network services outside of the one country.

In some embodiments, the at least one server 4 is configured to receive support data D4 from at least one external data source 41, 42, 43. Preferably, the recommendation module 4a is configured to determine the recommended course of action further based on the support data D4 received from the at least one external data source 41, 42, 43.

The support data D4 may be selected from the group including medical data, anonymous patient data (e.g., from other clinics/regions/countries), and third-party information. The medical data may be selected from the group including main indication, sub-indications, diagnosis, patient history, medication, laboratory results, and medical assessments. In some embodiments, at least a part of the medical data may be provided by an EHR (electronic health record) system. The third-party information may include weather, lifestyle, and the like.

The at least one server 4 and the at least one external data source 41, 42, 43 may be configured to communicate with each other via at least one communications network 12. In particular, the at least one server 4 may be configured to request the support data D4 from the at least one external data source 41, 42, 43. In response to the request, the at least one external data source 41, 42, 43 may transmit the support data D4 to the at least one server 4 via the at least one communications network 12.

Preferably, the at least one communications network 12 includes a local area network and/or a wide area network. For example, the wide area network may be configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), and Fifth Generation Technology Standard (5G).

In view of the above, the at least one server 4, such as a remote network service (RNS), may consider multiple data sources that may be of interest to support the user P. The at least one server 4 calculates and defines a recommendation for the user P using an artificial intelligence algorithm. The recommendation may be displayed on the external programming device 3, and the user P can decide whether to follow the recommendation or not. In the latter case, the user P may continue with the device testing. This makes it possible to optimize clinic workflow (reducing staff such as remote tech support or field service) and increase therapy quality and individualized care through additional computational effort, resulting in a positive therapy outcome and reduced clinic and healthcare costs.

Optionally, the at least one server 4 can be configured to calculate and display statistics and summaries of guidelines for similar indications and patient constellations (e.g., on the programming device 3). These guidelines may be official guidelines provided by national and international health organizations for various heart diseases/cardiac insufficiencies. The guidelines describe how an implant should be programmed for a particular indication. Furthermore, the user P can be given the possibility to search for similar indications and patient constellations in other clinics/regions/countries (anonymized data) and to find out how other physicians have programmed their patient devices.

The at least one server 4 includes the recommendation module 4a configured to provide the recommended course of action for the user P using the artificial intelligence algorithm based on the data D1 and/or the information D3 and/or the support data D4. To achieve sufficient sensitivity and specificity, the artificial intelligence algorithm can be trained with suitable training data, as will be explained in the following.

In one exemplary embodiment, the active medical implant 2 is configured for cardiac rhythm management. The training data can then include at least one of:
intracardiac or surface ECG for the detection of cardiac insufficiencies (e.g., atrial fibrillation)
measurement results of the implant (impedance, if necessary, shock impedance for defis, sensing, stimulus threshold)
program settings of the implant in the area of bradycardia
program settings of the implant in the area of tachycardia
general patient information (e.g., age, sex, weight, indication)
medication

In another exemplary embodiment, the active medical implant 2 is configured for spinal cord stimulation. The training data can then include at least one of:
general patient information (e.g., age, sex, weight, indication)
information of the used programs in the implant for therapy (e.g., electrode configuration, amplitude, pulse width, frequency, duty cycle, electrode offset, etc.)
information about how the programs are used by the user P (e.g., which program is used how often, amplitude settings)
motion profile via the sensor data of the implant
medication

The trained artificial intelligence algorithm can then determine the recommended course of action with sufficient sensitivity and specificity based on the data D1 and/or the information D3 and/or the support data D4 and send the recommended course of action to the programming device 3.

The programming device 3 may include a user interface, such as a touch user interface and/or a voice user interface, configured to output the recommended course of action to the user P. For example, the user interface may be configured to display information regarding the recommended course of action and/or output audio information regarding the recommended course of action.

In some embodiments, the user interface may be configured to receive a user input from the user P confirming the recommended course of action. Preferably, the programming device 3 is configured to automatically execute the recommended course of action in response to the confirmation received at the user interface ("one-click solution"). For example, the programming device 3 may be configured to automatically program the active medical implant 2 according to a recommended programming provided by the at least one server 4 in response to the confirmation received at the user interface.

According to some embodiments, the workflow of the system 1 may use a logically guided wizard or be based on the one-click solution described above. Furthermore, the execution of the recommended course of action can have human involvement (e.g., of the physician) or can be configured to be entirely automatic.

The embodiments of the present disclosure provide one or more of the following advantages:
optimization of clinic workflows (e.g., reduction of staff such as remote tech support or field service )
automatic AI/ML analysis of a large number of existing programs for different indications, patient (group) histories and/or other relevant events (complaints, studies, etc.) helps to identify more efficient/successful parameter combinations
while primary therapy settings are often the same for a larger number of patients, changing certain parameters leads to better personalized care (for example, an optimization of "night rate" parameters: analysis of patient history, e.g., blood pressure, heart rate, and/or medication, might show that slower pacing during the night is possible, leading to higher therapy and quality of life)
one-click programming (confirmation programs only) saves time for medical staff complex calculations, which require a lot of hardware resources, do not have to be performed on external programming devices, thus a significant cost reduction is possible calculations can be performed that are technically not possible on an external programming device

While the foregoing is directed to embodiments of the disclosure, other and further embodiments of the disclosure may be devised without departing from the basic scope thereof, and the scope thereof is determined by the claims that follow.

## Claims

1. A system (1) for programming an active medical implant (2) by means of a programming device (3), comprising:
a programming device (3) configured for programming an active medical implant (2); and
at least one server (4),
wherein the programming device (3) is configured to communicate with the at least one server (4) and to transmit to the server (4) data (D1) requested from the implant (2) and/or information (D3) provided by a user (P) regarding a patient and/or the implant (2), and
wherein the at least one server (4) includes a recommendation module (4a) configured to determine a recommended course of action for the user (P) using an artificial intelligence algorithm based on said data (D1) and/or information (D3), wherein the programming device (3) is configured to output the recommended course of action to the user (P).

2. The system (1) of claim 1, wherein the artificial intelligence algorithm is a machine learning algorithm, in particular a neural network, configured to determine the recommended course of action.

3. The system (1) of claim 1 or 2, wherein the programming device (3) includes a user interface, and wherein the user interface is configured to output the recommended course of action to the user (P).

4. The system (1) of claim 3, wherein the user interface is configured to receive a user input from the user (P) confirming the recommended course of action.

5. The system (1) of claim 4, wherein the programming device (3) is configured to automatically execute the recommended course of action in response to the confirmation received at the user interface.

6. The system (1) of any one of claims 1 to 5, wherein the recommended course of action includes a recommended programming of the active medical implant (2).

7. The system (1) of any one of claims 1 to 6, wherein the programming device (3) and the at least one server (4) are connected via at least one communications network (11), in particular a local area network and/or a wide area network.

8. The system (1) of claim 7, wherein the wide area network is configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), and Fifth Generation Technology Standard (5G).

9. The system (1) of any one of claims 1 to 8, wherein the programming device (3) and the at least one server (4) are configured for encrypted communication, in particular by means of at least one of symmetric encryption, asymmetric encryption, hashes, and signatures.

10. The system (1) of any one of claims 1 to 9, wherein the at least one server (4) is configured to receive support data (D4) from at least one external data source (41, 42, 43), wherein the recommendation module (4a) is configured to determine the recommended course of action further based on the support data (D4) received from the at least one external data source (41, 42, 43).

11. The system (1) of any one of claims 1 to 10, wherein the system (1) is configured for programming the active medical implant (2) during an implantation of the active medical implant (2) and/or a follow-up session after the implantation.

12. The system (1) of any one of claims 1 to 11, wherein the programming device (3) is a stationary or portable medical support device for medical personnel.

13. The system (1) of any one of claims 1 to 12, wherein the active medical implant (2) is configured for cardiac rhythm management or spinal cord stimulation.

14. A method for programming an active medical implant (2) by means of a programming device (3), comprising:
receiving, at a programming device (3) configured for programming an active medical implant (2), data (D1) from the implant (2) and/or information (D3) provided by a user (P) regarding the implant (2);
receiving, at a server (4), the data (D1) from the implant (2) and/or the information (D3) from the programming device (3);
determining, by the server (4), a recommended course of action for the user (P) using an artificial intelligence algorithm based on said data (D1) and/or information (D3); and
outputting, by the programming device (3), the recommended course of action to the user (P).

15. A machine-readable medium, comprising instructions executable by processors to implement the method for programming an active medical implant (2) by means of a programming device (3) of claim 13.
